# EUROPEAN PATENT SPECIFICATION

(11) **EP 3 510 141 B1**
(45) Date of publication and mention of the grant of the patent: **11.10.2023**
(21) Application number: 17768079.0
(22) Date of filing: 12.09.2017
(51) Int. Cl.: C12N 5/071, C12P 21/02, C07K 16/28, C12N 5/00, C12P 1/00

(54) **METHODS FOR MODULATING PRODUCTION PROFILES OF RECOMBINANT PROTEINS**
VERFAHREN ZUR MODULATION VON HERSTELLUNGSPROFILEN REKOMBINANTER PROTEINE
PROCÉDÉS DE MODULATION DE LA PRODUCTION DE PROFILS DE PROTÉINES DE RECOMBINAISON

(30) Priority: 12.09.2016 EP 16188411
(43) Date of publication of application: 17.07.2019
(73) Proprietor: Ares Trading S.A., 1170 Aubonne (CH)
(72) Inventor: BIELSER, Jean-Marc, 1616 Attalens (CH); DESMURGET, Caroline, 1814 La Tour-de-Peilz (CH)
(74) Representative: Merck Serono S.A. Intellectual Property
(86) International application number: PCT/EP2017/072936
(87) International publication number: WO 2018/046769

(56) References cited:
- WO-A1-02/077202
- JIN HYOUNG PARK ET AL: "Valeric acid induces cell cycle arrest at G1 phase in CHO cell cultures and improves recombinant antibody productivity", BIOTECHNOLOGY JOURNAL, vol. 11, no. 4, 1 April 2016 (2016-04-01), pages 487-496, XP055341970, DE ISSN: 1860-6768, DOI: 10.1002/biot.201500327
- CORONEL JULIANA ET AL: "Valeric acid supplementation combined to mild hypothermia increases productivity in CHO cell cultivations", BIOCHEMICAL ENGINEERING JOURNAL, ELSEVIER, AMSTERDAM, NL, vol. 114, 29 June 2016 (2016-06-29), pages 101-109, XP029735260, ISSN: 1369-703X, DOI: 10.1016/J.BEJ.2016.06.031

## Description

### FIELD OF THE INVENTION

The invention is in the field of cell culture. Particularly the field of the invention is the culturing of a host cell expressing a recombinant protein in a cell culture medium supplemented with feeds comprising effective amounts of valeric acid.

### BACKGROUND OF THE INVENTION

In the last 20 years, biopharma companies made lots of efforts trying to find compounds that could boost cell growth and productivity of recombinant proteins. Many authors reported the positive effect of small additives on specific productivity, often because they arrest cell culture in G0 [1], which is the most important phase for production. Amongst them, carboxylic acids have a large impact on culture behavior. They were found to inhibit HDAC1 activity (histone desacetylase 1) [2]. But adding chemicals to cultivation, even if it can increase titers, often leads to a reduced quality. These chemicals have also cytotoxic and apoptotic activities. Many approaches thus aimed at comparing different carboxylic acids and studying their effect on titer and quality to find the less detrimental for proteins. For instance, Park et al. (Biotechnol. J. 2016, 11, 487-496) used valeric acid in cell culture medium in the context of recombinant Chinese hamster ovary (CHO) cell culture for recombinant antibody manufacturing processes, and demonstrated that inhibited cell growth but increased mAb production, the highest "maximum mAb concentration " (MMC) was obtained when the valeric acid was added to the cell culture medium on day 0 of the cell culture, that is to say valeric acid was present in the medium at the start of the culture.

Therefore, there remains a need for culture conditions and production methods that allow safe culturing of host cells with increased production of recombinant proteins. The present invention addresses this need by providing methods and compositions for increasing production of a recombinant protein, while also improving the viability of the host cells.

### SUMMARY OF THE INVENTION

The invention is set out in the appended set of claims.

### BRIEF DESCRIPTION OF THE FIGURES

**Figure 1** shows the viable cell density (A); viability (B); and titer (C) for the host cells expressing protein P1 cultured with different concentrations of valeric acid and different timing for feeding, The legend for all of Figures 1A, 1B and 1C is reported in Figure 1D.
**Figure 2** shows the viable cell density (A); viability (B); and titer (C) for the host cells expressing antibody P2 cultured with 1.5 mM valeric acid added at different timing, The legend for all of Figures 2A, 2B and 2C is reported in Figure 2D

In both figures: VA = valeric acid and WD = working day (i.e. the day of culture on which the cell culture is fed with valeric acid)

### DETAILED DESCRIPTION OF THE INVENTION

Unless defined otherwise, all technical and scientific terms used herein have the same meaning as is commonly understood by one of skill in art to which the subject matter herein belongs. As used herein, the following definitions are supplied in order to facilitate the understanding of the present invention. The term "comprise" is generally used in the sense of include, that is to say permitting the presence of one or more features or components. Also as used in the specification and claims, the language "comprising" can include analogous embodiments described in terms of "consisting of "and/or "consisting essentially of'.

As used in the specification and claims, the singular form "a", "an" and "the" include plural references unless the context clearly dictates otherwise. The term "and/or" used in a phrase such as "A and/or B" herein is intended to include "A and B", "A or B", "A", and "B".

The term "cell culture" or "culture" is meant the growth and propagation of cells in vitro, i.e. outside of an organism or tissue. Suitable culture conditions for mammalian cells are known in the art, such as taught in Cell Culture Technology for Pharmaceutical and Cell-Based Therapies (2005)[3]. Mammalian cells may be cultured in suspension or while attached to a solid substrate.

The terms "cell culture medium," "culture medium", "medium," and any plural thereof, refer to any medium in which cells of any type can be cultured. A "basal medium" refers to a cell culture medium that contains all of the essential ingredients useful for cell metabolism. This includes for instance amino acids, lipids, carbon source, vitamins and mineral salts. DMEM (Dulbeccos' Modified Eagles Medium), RPMI (Roswell Park Memorial Institute Medium) or medium F12 (Ham's F12 medium) are examples of commercially available basal media. Alternatively, said basal medium can be a proprietary medium fully developed in-house, also herein called "chemically defined medium" or "chemically defined culture medium", in which all of the components can be described in terms of the chemical formulas and are present in known concentrations. The culture medium can be free of proteins and/or free of serum, and can be supplemented by any additional compound(s) such as amino acids, salts, sugars, vitamins, hormones, growth factors, depending on the needs of the cells in culture.

The term "feed medium" or "feed" (and plural thereof) refers to a medium (or to a composition) used as a supplementation during culture to replenish the nutrients which are consumed and/or to supply additional nutrients and/or additional compounds needed during cell culture (for example to enhance or improve cell growth and production of recombinant protein). The feed medium can be a commercially available feed medium or a proprietary feed medium (herein alternatively chemically defined feed medium). When only specific additional compounds, such as valeric acid, have to be supplied to the culture media, the feed can comprise only this compound, preferably in a liquid form.

The term "bioreactor" or "culture system" refers to any system in which cells can be cultured, such as in perfusion, batch or fed-batch mode. This term includes but is not limited to flasks, static flasks, spinner flasks, tubes, shake tubes, shake bottles, wave bags, bioreactors, fiber bioreactors, fluidized bed bioreactors, and stirred-tank bioreactors with or without microcarriers. Alternatively, the term "culture system" also includes microtiter plates, capillaries or multi-well plates. Any size of bioreactor can be used, for instance from 0.1 milliliter (0.1 mL, very small scale) to 20000 liters (20000L or 20 KL, large scale), such as 0.1 mL, 0.5 mL 1 mL, 5 mL, 0.01L, 0.1L, 1L, 2L, 5L, 10L, 50L, 100L, 500L, 1000L (or 1KL), 2000L (or 2K), 5000L (or 5KL), 10000L (or 10KL), 15000L (or 15KL) or 20000L (20KL).

The term "fed-batch culture" refers to a method of growing cells, where there is a bolus or continuous feed media supplementation to replenish the nutrients which are consumed and/or supplementation of additional nutrients and/or additional compounds needed during cell culture (for example to enhance or improve cell growth and production of recombinant protein). This cell culture technique has the potential to obtain high cell densities in the order of greater than 10 × 10⁶ to 30 × 10⁶ cells/ml, depending on the media formulation, cell line, and other cell growth conditions. A biphasic culture condition can be created and sustained by a variety of feed strategies and media formulations.

Alternatively a perfusion culture can be used. Perfusion culture is one in which the cell culture receives fresh perfusion feed medium while simultaneously removing spent medium. Perfusion can be continuous, step-wise, intermittent, or a combination of any or all of any of these. Perfusion rates can be less than a working volume to many working volumes per day. Preferably the cells are retained in the culture and the spent medium that is removed is substantially free of cells or has significantly fewer cells than the culture. Perfusion can be accomplished by a number of cell retention techniques including centrifugation, sedimentation, or filtration [4].

When using the methods and/or cell culture techniques of the instant invention, the recombinant proteins are generally directly secreted into the culture medium. Once said proteins are secreted into the medium, supernatants from such expression systems can be first concentrated using a commercially available protein concentration filter.

As used herein, "cell density" refers to the number of cells in a given volume of culture medium. "Viable cell density" refers to the number of live cells in a given volume of culture medium, as determined by standard viability assays. The cell density will be considered as maintained if it is in the range of about -10% to +10% compared to the control culture condition (i.e. cells grown with feeds comprising no valeric acid or any other inducer).

The term "viability", or "cell viability" refers to the ratio between the total number of viable cells and the total number of cells in culture. Viability is usually acceptable as long as it is at not less than 60 % compared to the start of the culture (however, the acceptable threshold can be determined case by case). Viability is often used to determine time for harvest. For instance, in fed-batch culture, harvest can be performed once viability reaches at 60% or after 14 days in culture.

The wording "titre" refers to the amount or concentration of a substance, here the recombinant protein of interest, in solution. It is an indication of the number of times the solution can be diluted and still contain detectable amounts of the molecule of interest. It is calculated routinely for instance by diluting serially (1:2, 1:4, 1:8, 1:16, etc) the sample containing the protein of interest and then using appropriate detection method (colorimetric, chromatographic etc.), each dilution is assayed for the presence of detectable levels of the protein of interest. Titre can also be measured by means such as by fortéBIO Octet^{®} or with Biacore C^{®}, as used in the example section, or by any other means known by the skilled person.

The terms "higher titre" or "increased production" and equivalents thereof, means that the titre is increased by at least 10% when compared to the control culture condition. The titre will be considered as maintained if it is in the range of -10% to 10% compared to the control culture condition. The terms "lower titre" and equivalents thereof, means that the titre is decreased by at least 10% when compared to the control culture condition (i.e. cells grown with feeds comprising no valeric acid or any other inducer).

The term "protein" as used herein includes peptide and polypeptide and refers to compound comprising two or more amino acid residues. A protein according to the present invention includes but is not limited to a cytokine, a growth factor, a hormone, a fusion protein, an antibody or a fragment thereof. A therapeutic protein refers to a protein that can be used or that is used in therapy.

The term "recombinant protein" means a protein produced by recombinant technics. Recombinant technics are well within the knowledge of the skilled person [5].

The term "antibody", and its plural form "antibodies", includes, inter alia, polyclonal antibodies, affinity-purified polyclonal antibodies, monoclonal antibodies, and antigen-binding fragments, such as F(ab')2, Fab proteolytic fragments, and single chain variable region fragments (scFvs). Genetically engineered intact antibodies or fragments, such as chimeric antibodies, humanised antibodies, human or fully human antibodies, scFv and Fab fragments, as well as synthetic antigen-binding peptides and polypeptides, are also included.

The term "humanized" immunoglobulin (or "humanised antibody) refers to an immunoglobulin comprising a human framework region and one or more CDRs from a non-human (usually a mouse or rat) immunoglobulin. The non-human immunoglobulin providing the CDRs is called the "donor" and the human immunoglobulin providing the framework is called the "acceptor" (humanization by grafting non-human CDRs onto human framework and constant regions, or by incorporating the entire non-human variable domains onto human constant regions (chimerization)). Constant regions need not be present, but if they are, they must be substantially identical to human immunoglobulin constant regions, i.e., at least about 85-90%, preferably about 95% or more identical. Hence, all parts of a humanized immunoglobulin, except possibly the CDRs and a few residues in the heavy chain constant region if modulation of the effector functions is needed, are substantially identical to corresponding parts of natural human immunoglobulin sequences. Through humanizing antibodies, biological half-life may be increased, and the potential for adverse immune reactions upon administration to humans is reduced. The term "fully human" immunoglobulin (or "fully-human" antibody) refers to an immunoglobulin comprising both a human framework region and human CDRs. Constant regions need not be present, but if they are, they must be substantially identical to human immunoglobulin constant regions, i.e., at least about 85-90%, preferably about 95% or more identical. Hence, all parts of a fully human immunoglobulin, except possibly few residues in the heavy chain constant region if modulation of the effector functions or pharmacokinetic properties are needed, are substantially identical to corresponding parts of natural human immunoglobulin sequences. In some instances, amino acid mutations may be introduced within the CDRs, the framework regions or the constant region, in order to improve the binding affinity and/or to reduce the immunogenicity and/or to improve the biochemical/biophysical properties of the antibody.

The term "recombinant antibody" (or "recombinant immunoglobulin) means antibody produced by recombinant technics. Because of the relevance of recombinant DNA techniques in the generation of antibodies, one needs not be confined to the sequences of amino acids found in natural antibodies; antibodies can be redesigned to obtain desired characteristics. The possible variations are many and range from the changing of just one or a few amino acids to the complete redesign of, for example, the variable domain or constant region. Changes in the constant region will, in general, be made in order to improve, reduce or alter characteristics, such as complement fixation (e.g. complement dependent cytotoxicity, CDC), interaction with Fc receptors, and other effector functions (e.g. antibody dependent cellular cytotoxicity, ADCC), pharmacokinetic properties (e.g. binding to the neonatal Fc receptor; FcRn). Changes in the variable domain will be made in order to improve the antigen binding characteristics. In addition to antibodies, immunoglobulins may exist in a variety of other forms including, for example, single-chain or Fv, Fab, and (Fab')2 , as well as diabodies, linear antibodies, multivalent or multispecific hybrid antibodies.

The term "antibody portion" refers to a fragment of an intact or a full-length chain or antibody, usually the binding or variable region. Said portions, or fragments, should maintain at least one activity of the intact chain / antibody, i.e. they are "functional portions" or "functional fragments". Should they maintain at least one activity, they preferably maintain the target binding property. Examples of antibody portions (or antibody fragments) include, but are not limited to, "single-chain Fv", "single-chain antibodies," "Fv" or "scFv". These terms refer to antibody fragments that comprise the variable domains from both the heavy and light chains, but lack the constant regions, all within a single polypeptide chain. Generally, a single-chain antibody further comprises a polypeptide linker between the VH and VL domains which enables it to form the desired structure that would allow for antigen binding. In specific embodiments, single-chain antibodies can also be bi-specific and/or humanized.

A "Fab fragment" is comprised of one light chain and the variable and CH1 domains of one heavy chain. The heavy chain of a Fab molecule cannot form a disulfide bond with another heavy chain molecule. A "Fab' fragment" that contains one light chain and one heavy chain and contains more of the constant region, between the CH1 and CH2 domains, such that an interchain disulfide bond can be formed between two heavy chains is called a F(ab')2 molecule. A "F(ab')2" contains two light chains and two heavy chains containing a portion of the constant region between the CH1 and CH2 domains, such that an interchain disulfide bond is formed between two heavy chains. Having defined some important terms, it is now possible to focus the attention on particular embodiments of the instant invention.

Examples of known antibodies which can be produced according to the present invention include, but are not limited to, adalimumab, alemtuzumab, belimumab, bevacizumab, canakinumab, certolizumab pegol, cetuximab, denosumab, eculizumab, golimumab, infliximab, natalizumab, ofatumumab, omalizumab, pertuzumab, ranibizumab, rituximab, siltuximab, tocilizumab, trastuzumab, ustekinumab or vedolizomab.

The terms "Inducing agent", "inducer" or "productivity enhancer" refer to a compound allowing an increase of the protein production when added in cell cultures. For instance, one of the inducers known for E.coli production is IPTG (Isopropyl β-D-1-thiogalactopyranoside) and inducers for CHO production are among others sodium butyrate, doxycycline or dexamethasone.

The term "subject" is intended to include (but not limited to) mammals such as humans, dogs, cows, horses, sheep, goats, cats, mice, rabbits, or rats. More preferably, the subject is a human.

The present disclosure presents methods seeking to increase production of a recombinant protein while avoiding decrease in cell viability over a production period. The present invention is based on the optimisation of cell culture conditions for protein manufacturing, such as production of antibodies or antigen-binding fragments, resulting in increased production of a recombinant protein while avoiding decrease in cell viability over a production period.

The inventors have surprisingly found that under cell culture conditions supplemented during the culture process with valeric acid, the production of a recombinant protein can be increased (i.e. the titre is increased), and substantial or significant decrease in cell viability over a production period is avoided. Thus during the cell culture production run, when it is desirable to increase titre of a recombinant protein being produced, the cell culture can be supplemented with at least one feed comprising or consisting of valeric acid. Indeed, cell cultures supplemented with valeric acid in at least one feed, at different concentrations and timings, provided (1) significantly increased percentage of G0/G1 cells, and (2) increased titer", compare to a similar cell culture not supplemented with valeric acid.

Valeric acid, or pentanoic acid, is a straight-chain alkyl carboxylic acid with the chemical formula C₅H₁₀O₂.

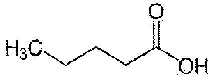

In one aspect the invention provides a method for the production of a recombinant protein, said method comprising culturing a mammalian host cell expressing said recombinant protein in cell culture medium supplemented, on day 5 after the start of the culture, with at least one feed comprising an effective amount of valeric acid, wherein the concentration of the valeric acid supplement in the cell culture medium is about 1 mM to 2 mM.

The term "effective amount of valeric acid" corresponds to the amount of valeric acid added to a cell culture (or a cell culture medium) as a supplement (i.e. a feed consisting only of valeric acid in a liquid form) or as a feed component (i.e. together with nutrients needed for cell culture), that will increase expression of the recombinant protein in host cells, and possibly also increase or at least maintain cell viability, by a detectable amount when compared to similar host cells grown without valeric acid in the feeds. Valeric acid is not present in nor added to a cell culture medium at the start of the culture. Valeric acid is preferably added to a cell culture (or a cell culture medium), as a supplement or as a feed component, at a concentration of about 1.5 mM. In some embodiments, the concentration of valeric acid can be for instance of about 1 mM, 1.1 mM, 1.2 mM, 1.3 mM, 1.4 mM, 1.5 mM, 1.6 mM, 1.7 mM, 1.8 mM, 1.9mM, 2.0 mM(concentration of valeric acid once in the culture medium in the culture system). For example, but not by way of limitation, by adjusting the concentration of valeric acid, the production of secreted recombinant protein can be modulated (i.e. increased).

As used herein, the phrase "cell viability does not substantially or significantly decrease", when compared to cells grown without valeric acid or any other inducer, means that cell viability does not decrease more than about 15% compared to control cultures (i.e. cells grown with feed comprising no valeric acid or any other inducer).

For the purposes of this invention, cell culture medium is a medium suitable for growth of animal cells, such as mammalian cells, in *in vitro* cell culture. Cell culture media formulations are well known in the art. Cell culture media may be supplemented with additional components such as amino acids, salts, sugars, vitamins, hormones, and growth factors, depending on the needs of the cells in culture.

Preferably, the cell culture media are free of animal components; they can be serum- free and/or protein-free.

In the context of the invention as a whole, the cell culture medium is supplemented with valeric acid in a fed-batch or in a continuous manner, preferably in a fed-bactch manner. The addition of valeric acid supplement may be based on measured intermediate titre.

In an embodiment of the present invention as a whole, the host cell is a mammalian host cell (herein also refer to as a mammalian cell) including, but not limited to, HeLa, Cos, 3T3, myeloma cell lines (for instance NS0, SP2/0), and Chinese hamster ovary (CHO) cells, such as CHO-S cell and CHO-k1 cell. In a preferred embodiment, the host cell is a Chinese Hamster Ovary (CHO) cell, such as CHO-S cell and CHO-k1 cell.

In the context of the invention as a whole, the recombinant mammalian cell, is grown in a culture system such as a bioreactor. The bioreactor is inoculated with viable cells in a culture medium. Said culture medium is supplemented with valeric acid after the start of the culture, preferably when the cells have reached the stationary phase. Preferably the culture medium is serum free and/or protein-free. Once inoculated into the production bioreactor, the recombinant cells undergo an exponential growth phase. The stationary phase (i.e. production phase) can be maintained using a fed-batch process with bolus feed(s) of a feed medium supplemented with valeric acid or of a feed consisting only of valeric acid in a liquid form. Preferably the feed is serum-free and/or protein-free. The supplemental bolus feeds typically begin shortly after the cells are inoculated into the bioreactor, at a time when it is anticipated or determined that the cell culture needs feeding. For example, supplemental feeds comprising or consisting of valeric acid can begin on or around day 6 or 7 of the culture. The culture may receive one, two, three, or more bolus feeds comprising or consisting of valeric acid during the growth phase. The supplementation with valeric acid can be done in fed-batch, and/or in continuous manner. The culture medium can comprise a sugar, such as glucose or be supplemented by a sugar, such as glucose. Said supplementation in sugar can be done at the start of the culture, in fed-batch, and/or in continuous manner.

The method according to the present invention may be used in the present disclosure not part of the invention to improve the production of recombinant proteins in multistep culture processes. In a multiple stage process, cells are cultured in two or more distinct phases. For example cells are cultured first in one or more growth phases, under conditions improving cell proliferation and viability, then transferred to production phase(s), under conditions improving protein production. In a multistep culture process, some conditions may change from one step (or one phase) to the other: media composition, shift of pH, shift of temperature, etc. The growth phase can be performed at a temperature higher than in production phase. For example, the growth phase can be performed at a first temperature from about 35°C to about 38°C, and then the temperature is shifted for the production phase to a second temperature from about 29°C to about 37°C, preferably from about 34°C to about 36.5°C. The cell cultures can be maintained in production phase for days or even weeks before harvest.

The cell lines (also referred to as "recombinant cells" or "host cells") used in the invention are genetically engineered to express a protein of commercial or scientific interest. Methods and vectors for genetically engineering of cells and/or cell lines to express a polypeptide of interest are well known to those of skill in the art; for example, various techniques are illustrated in Ausubel et al. (1988, and updates; [6]) or Sambrook et al. (1989, and updates; [5]). The methods of the invention can be used in the present disclosure not part of the invention to culture cells that express recombinant proteins of interest. The recombinant proteins are usually secreted into the culture medium from which they can be recovered. The recovered proteins can then be purified, or partially purified using known processes and products available from commercial vendors. The purified proteins can then be formulated as pharmaceutical compositions. Suitable formulations for pharmaceutical compositions include those described in Remington's Pharmaceutical Sciences (1995 and updated; [7]). In the context of the invention as a whole, the recombinant protein is selected from the group consisting of an antibody or antigen binding fragment thereof, such as a human (or fully human) antibody or antigen-binding portion thereof, a humanised antibody or antigen-binding portion thereof, a chimeric antibody or antigen-binding portion thereof, a fusion protein, a growth factor, a hormone, or a cytokine. Those skilled in the art will appreciate that the invention described herein is susceptible to variations and modifications other than those specifically described.

The foregoing description will be more fully understood with reference to the following examples.

### EXAMPLES

### Material and methods

### I. Cells, cell expansion and cell growth

### 1) Cells

Assays were performed with 2 CHO cell lines:
- CHO-S cells expressing a fusion protein P1, herein "Cells P1" or "P1 cells". "P1" is an IgG1 fusion protein, comprising one part directed against a membrane protein (IgG part) linked to a second part targetting a soluble immune protein. Its isoelectric point (pl) is about 6.3-7.0.
- CHO-K1 cells expressing a monoclonal antibody P2, herein "Cells P2" or "P2 cells". "P2" is a humanised monoclonal antibody directed against a receptor found on the cell membrane. Its isoelectric point (pl) is about 9.20-9.40.

### 2) Cell expansion

Cell expansion was performed in tubes in a medium suitable for cell expansion. Assays in fed-batch started after at least one week expansion.

### 3) Inoculation

Cells expressing both P1 and P2 were inoculated at 0.2 x 10⁶ viable cells per millilitre (mL).

### 4) Fed-batch conditions

All assays were performed in fed-batch culture. The host cells were cultured in fed-batch system either in microplates ("Deep well plate", "DWP") or in Spin tubes^{®}, and incubated at 36.5°C, 90% relative humidity, 5% CO₂ and 320rpm shaking during 14 days.

A stock solution of valeric acid was prepared in a standard production proprietary medium. The valeric acid concentration of this stock solution was 195 mM. The stock solution was used to supplement media to test different valeric acid concentrations from the beginning of the culture (day 0; tested concentrations: 1, 1.5 and 2 mM). The solution was also used like a feed to supplement the valeric acid to the culture at different time points during the fed-batch (day 3, 4, 5 or 7). When the solution was used like a feed, the target concentration in the culture was always 1.5 mM.

### II. Analytical methods

Viable cell density and viability were measured with the Guava easyCyte^{®} flow cytometer or with the ViCell. Antibody titres were measured with the fortéBIO Octet^{®} or with Biacore C^{®}.

### Results

### a. Experiences with P1 cells

To better understand the potential of valeric acid for fed-batch cultivations, supplementation in media (at time "0") or separately in feeds (at different time points, i.e. day 3, 4, 5 or 7) were tested. Cell growth was significantly impacted.

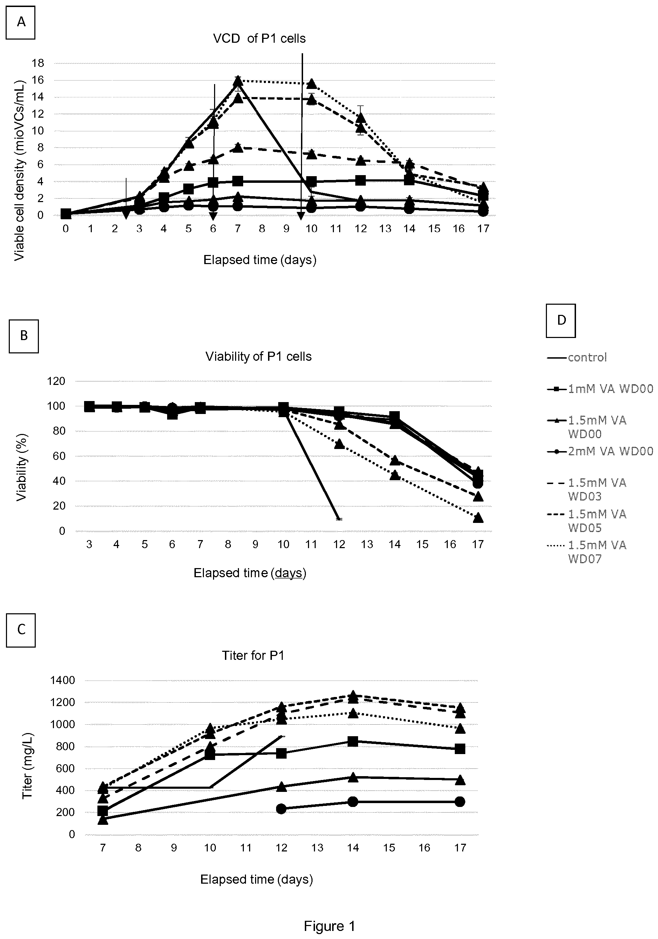

Different concentrations were used on day 0. The higher the concentration, the lower the maximum viable cell density (Figure 1A). The viability was maintained longer than for control experiment, but because of the low cell density the final titer was not improved (Figures 1B and 1C).

When valeric acid was added at different feeding days, a clear impact was visible already the next day. Cell growth rate was slowed down when valeric acid was added at day 3, but in overall maintain when valeric acid was added at days 5 or 7, compared to control (Figure 1A). However as shown in figure 1B, this had no impact on cell viability until day 11. As of day 12, the viability of the culture was prolonged compared to the control runs. As shown in Figure 1C, although the cell growth rate when valeric acid was added at day 3 was lower than control, the titer was positively impacted by the addition of valeric acid (figure 1C). This positive impact was observed whatever the day at which valeric acid was added. At day 12 for instance, the increase of titer was about 20% for valeric acid at day 3 or 7 and about 30% for valeric acid at day 5, compared to control. The cell diameter seemed also to be impacted (data not shown) and was linked to specific productivity. If cells were favored to stay in a G0/G1 state it is probable that they direct more of their energy consumption towards protein production.

In this experiment it seemed clear that the addition of valeric acid in the media on day 0 did not improve the productivity of the process. To the contrary, addition of valeric acid at this point of time had a very negative impact on both cell density and titer. Regarding the addition as a feed, the time of addition seemed to play an important role and was further investigated.

### b. Experiences with P2 cells

In this experiment the addition of 1.5 mM valeric acid to the culture on day 03, 04 or 05 was tested. For this experiment a CHO-k1 cell line (P2 cells) was used to confirm that valeric acid has the same effect than on CHO-S cells (P1 cells as discussed in a.).

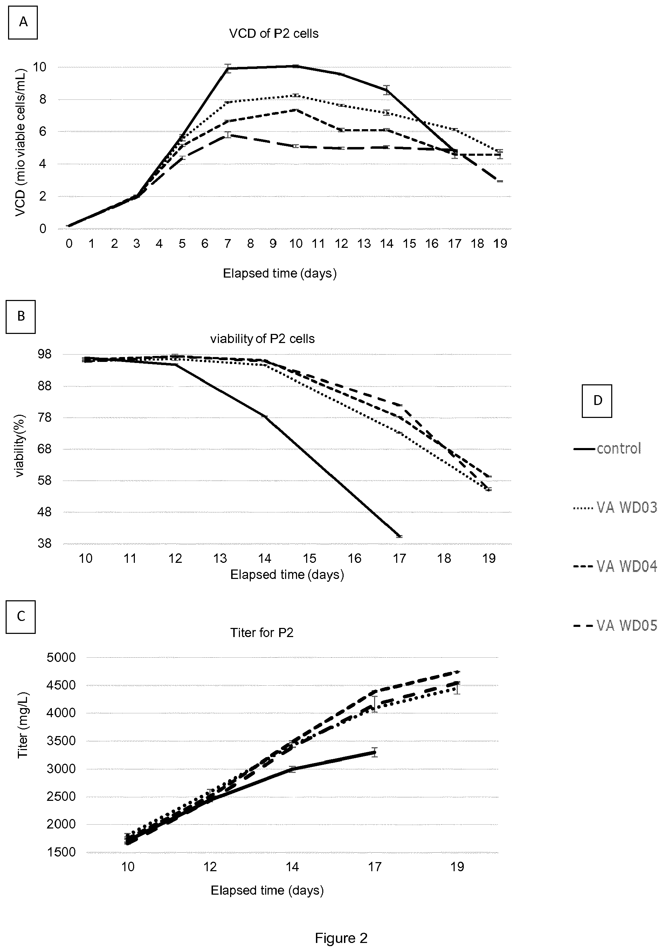

The maximum viable cell density decreased when the valeric acid was added earlier during the culture, compared to control (Figure 2A). However, this had no impact on cell viability, compared to control (Figure 2B). As for the previous experience with P1 cells, the viability was maintained compared to control until day 12 and was then prolonged compared to the control culture, starting day 13. This observation was independent of the valeric acid addition timing. In addition, although the titer was comparable to control until day 12, starting day 13, the titer continued to increase compared to control (figure 2C). For instance, at day 17, the titer was increased by about 25% for valeric acid at day 3 or 7 and about 35% for valeric acid at day 5, compared to control. The result was that cells were able to produce for a longer period and the run could potentially be further extended (up to day 20). In this example the productivity improvement became significant after day 17 and almost 5 g/L were obtained with the optimal condition, instead of 0.9 g/L for the control culture without valeric acid (data not shown).

### Conclusions

The inventors have shown that the addition of valeric acid in the media on day 0 did not improve the productivity of the process. To the contrary, addition of valeric acid at this point of time had a very negative impact on both cell density and titer. However, surprisingly they have shown that when valeric acid was added as a feed at day 3 or after, the titer can be increased, whatever the cell line or the molecule to be produced. As the viability is prolonged, the cells are able to produce for a longer period, even more increasing the level of production of protein, such as antibodies. The exact concentration of valeric acid to be added in the cell culture media will have to be determined case by case, although 1.5 mM seems very promising. This determination can be done without involving any inventive skill, based on the teaching of the present invention. The skilled person will also understand that he can use valeric acid in any method for producing any protein such as an antibody or fusion proteins.

### REFERENCES

[1] M. Butler, "Animal cell cultures: recent achievements and perspectives in the production of biopharmaceuticals," pp. 283-291, 2005.
[2] G. Bora-tatar, D. Dayangaç-erden, A. S. Demir, S. Dalkara, and K. Yelekçi, "Bioorganic & Medicinal Chemistry Molecular modifications on carboxylic acid derivatives as potent histone deacetylase inhibitors: Activity and docking studies," Bioorg. Med. Chem., vol. 17, no. 14, pp. 5219-5228,2009.
[3] Cell Culture Technology for Pharmaceutical and Cell-Based Therapies, Sadettin Ozturk, Wei-Shou Hu, ed., CRC Press (2005)
[4] Voisard et al., 2003, Biotechnol. Bioeng. 82:751-765
[5] Sambrook et al., 1989 and updates, Molecular Cloning: A Laboratory Manual, Cold Spring Laboratory Press
[6] Ausubel et al., 1988 and updates, Current Protocols in Molecular Biology, eds. Wiley & Sons, New York
[7] Remington's Pharmaceutical Sciences, 1995, 18th ed., Mack Publishing Company, Easton, PA

## Claims

1. A method for the production of a recombinant protein, said method comprising culturing a mammalian host cell expressing said recombinant protein in cell culture medium supplemented, on day 5 after the start of the culture, with at least one feed comprising an effective amount of valeric acid, wherein the concentration of the valeric acid supplement in the cell culture medium is about 1 mM to 2 mM.

2. The method according to claim 1, wherein the mammalian cell is Chinese Hamster Ovary (CHO) cell.

3. The method according to claims 1 or 2, wherein the recombinant protein is selected from the group consisting of an antibody or antigen binding fragment thereof, such as a human antibody or antigen-binding portion thereof, a humanised antibody or antigen-binding portion thereof, a chimeric antibody or antigen-binding portion thereof, a recombinant fusion protein, a growth factor, a hormone, or a cytokine.

4. The method according to any one of claims 1 to 3, wherein the concentration of the valeric acid supplement in the cell culture medium is about 1.5 mM.

## Patentansprüche

1. Verfahren zur Herstellung eines rekombinanten Proteins, wobei das Verfahren das Züchten einer Säugetier-Wirtszelle, die das rekombinante Protein exprimiert, in einem Zellkulturmedium umfasst, das am Tag 5 nach dem Beginn der Kultur mit mindestens einem Futtermittel ergänzt wird, das eine wirksame Menge an Valeriansäure umfasst, wobei die Konzentration des Valeriansäure-Zusatzes in dem Zellkulturmedium etwa 1 mM bis 2 mM beträgt.

2. Verfahren nach Anspruch 1, wobei es sich bei der Säugetierzelle um eine Ovarialzelle des Chinesischen Hamsters (CHO) handelt.

3. Verfahren nach Anspruch 1 oder 2, wobei das rekombinante Protein aus der Gruppe ausgewählt ist, die aus einem Antikörper oder einem antigenbindenden Fragment davon, wie einem menschlichen Antikörper oder einem antigenbindenden Teil davon, einem humanisierten Antikörper oder einem antigenbindenden Teil davon, einem chimären Antikörper oder einem antigenbindenden Teil davon, einem rekombinanten Fusionsprotein, einem Wachstumsfaktor, einem Hormon oder einem Zytokin besteht.

4. Verfahren nach einem der Ansprüche 1 bis 3, wobei die Konzentration des Valeriansäure-Zusatzes im Zellkulturmedium etwa 1,5 mM beträgt.

## Revendications

1. Procédé de production d'une protéine de recombinaison, lequel procédé comporte le fait de cultiver une cellule hôte mammalienne exprimant ladite protéine de recombinaison dans un milieu de culture cellulaire auquel est ajouté en supplément, le cinquième jour après le démarrage de la culture, au moins un aliment comprenant de l'acide valérique en quantité efficace, et dans lequel procédé la concentration du supplément acide valérique dans le milieu de culture cellulaire vaut à peu près de 1 mM à 2 mM.

2. Procédé conforme à la revendication 1, dans lequel la cellule mammalienne est la cellule d'ovaire de hamster chinois (CHO).

3. Procédé conforme à la revendication 1 ou 2, dans lequel la protéine de recombinaison est choisie dans l'ensemble constitué par un anticorps ou un fragment d'anticorps se liant à l'antigène, comme un anticorps humain ou sa partie se liant à l'antigène, un anticorps humanisé ou sa partie se liant à l'antigène, ou un anticorps chimérique ou sa partie se liant à l'antigène, une protéine de recombinaison par fusion, un facteur de croissance, une hormone, et une cytokine.

4. Procédé conforme à l'une des revendications 1 à 3, dans lequel la concentration du supplément acide valérique dans le milieu de culture cellulaire vaut à peu près 1,5 mM.
